# EUROPEAN PATENT APPLICATION

(11) **EP 1 482 308 A1**
(43) Date of publication of application: **01.12.2004**
(21) Application number: 03737501.1
(22) Date of filing: 06.02.2003
(51) Int. Cl.: G01N 33/02, C12Q 1/04

(54) **METHOD OF EVALUATING QUALITIES OF FOOD OR DRINK AND INDICATOR THEREFOR**

(30) Priority: 06.02.2002 JP 2002029270; 10.06.2002 JP 2002168049
(71) Applicant: National Food Research Institute, Tsukuba-shi, Ibaraki 305-8642 (JP); Taisei Lamick Co. Ltd., Minamisaitama-gun, Saitama 349-0203 (JP)
(72) Inventor: ISSHIKI, Kenji, c/o National Food Research Inst., Tsukuba-shi, Ibaraki 305 -8642 (JP); OGAWA, Junzo, Chiba-shi, Chiba 262-0033 (JP)
(74) Representative: Grünecker, Kinkeldey, Stockmair & Schwanhäusser Anwaltssozietät
(86) International application number: PCT/JP2003/001222
(87) International publication number: WO 2003/067254

(57) **Abstract**

There are provided a method of evaluating a quality of foods and drinks and an indicator therefor in which the quality of the foods and drinks, particularly the lowering of freshness, i.e. an amount of microorganism proliferated in the foods and drinks can be detected simply and accurately to judge the quality. That is, an aerogen consisting of at least one of yeast, mold and bacteria is sealed together with a fermentation substrate in a closed transparent vessel or transparent soft film bag of a synthetic resin and a quality of foods and drinks is judged by an amount of a gas generated through the formation of an acid generated in the vessel (bag), and there is used an indicator for evaluating thereof.

## Description

### TECHNICAL FIELD

This invention relates to a method of objectively evaluating and evaluating a quality of goods, for example, fresh foods such as farm products, livestock products, fishery products and the like; processed foods such as lunches, dishes and the like; drinks such as juices, alcohols and the like; seasonings such as sources and the like; medicines such as vaccines and the like; biochemical samples, cosmetics and so on, particularly a change in quality or a drop of freshness of these goods resulted from an influence of environmental temperature or time passage as well as an indicator effective for use in this method.

### BACKGROUND ART

In the fresh goods, processed foods, drinks such as juices and the like, vaccines, bloods and so on (which are abbreviated as a general term "foods and drinks" hereinafter for convenience' sake), it is important to control a time kept in a distribution way, i.e. a passage of time but also a temperature history at the distribution environment in order to ensure the safety thereof. If the control of the environmental temperature or the time passage is mistaken, for example, the fresh foods bring about the change of quality (drop of freshness) but also may become putrefied and further cause the risk of generating food poisoning.

Such a quality change or putrefaction of the foods and drinks is mostly caused due to the fact that microorganisms proliferate over a period from the production through the distribution to consumers for eating and drinking. The quality of the foods and drinks is common to be subjectively judged by human's feeling on abnormal odor, color change, abnormal flavor and the like accompanied with the putrefaction of the foods and drinks themselves. However, there is individual difference in such a subjective judgment, so that it is actually difficult to know a right quality (proceeding degree of quality change or putrefaction).

Also, the drop of the quality in the foods and drinks may be caused even if the foods and drinks are stored in a chilled region (-5 to 5°C) or a cooling region (5 to 10°C). This is due to the fact that conditions of exposing the foods and drinks to a temperature environment or handling them, for example, time required for placing the foods and drinks into a cold insulation tank, number of putting in or out differ on the way of the actual distribution. Particularly, the putrefaction of the foods and drinks easily proceeds as the environmental temperature becomes higher or as the storing time becomes longer. Under such an environment, it is strongly demanded to develop an indicator for evaluating the drop of quality in the foods and drinks accompanied with the temperature rise or the passage of the preservation time in the course of the distribution.

As such an indicator for evaluating the quality drop, accident or the like of the foods and drinks, there is a method disclosed, for example, in JP-A-11-194053. This technique is a method wherein a temperature history is confirmed by diffusing and penetrating a diffusable dyestuff into a dyestuff diffusing layer to cause a color change by the temperature rise and the time passage. Also, JP-A-11-296086 discloses a method wherein a temperature history of the foods and drinks is indicated by using an ink changing color dependent upon the heating temperature and time, and directly printing a symbol, figure or letter on a package for the foods and drinks or attaching a printed paper or resin sheet onto the package. However, these methods are a method of presuming a degree of proliferation of microorganisms in the foods and drinks from a relation between the heating temperature and the holding time for the proliferation, and can not objectively judge what extent the microorganisms actually proliferate to. Therefore, the handling of the foods and drinks in the actual distribution course is common to be a disposal for avoiding risk even if the quality is still sufficiently maintained at a high level.

In the light of the aforementioned problems inherent to the conventional technique, it is an object of the invention to provide a method of evaluating the quality of the foods and drinks in which the degree of quality change or the drop of freshness can be judged simply and accurately by visually catching it as a degree of proliferation of microorganisms when the quality of the foods and drinks is changed by the variation of the temperature and/or the passage of time as well as an indicator therefor.

### DISCLOSURE OF THE INVENTION

The inventors have aimed at microorganisms derived from the foods included in the actually distributed foods and made various studies in order to achieve the above object. As a result, it has been found out that an aerogen having a gas producing function is effective for realizing the above object among the microorganisms and it is possible to objectively (visually) judge a quality of the foods and drinks (freshness, degree of quality change) by an amount of gas produced from the aerogen. That is, the invention is a method of evaluating a quality of foods and drinks, characterized in that a microorganism of a nature producing a gas, i.e. an aerogen is sealed in a closed transparent vessel or transparent soft film bag of a synthetic resin and a quality of foods and drinks to be judged is judged by an amount of gas produced from a substrate containing the aerogen (e.g. fermentation substrate).

Moreover, the aerogen is any one of yeast, mold and bacterium, and produces CO₂ and H₂ accompanied with the formation of an acid mainly from a carbohydrate above a temperature starting the proliferation. Also, components such as the foods and drinks to be judged and the microorganism derived from the foods and drinks (aerogen) are sealed in the vessel or bag. Further, a standard bubble size indicating a degree of quality is previously printed on a surface of the vessel or soft film bag of the synthetic resin. The standard bubble size printed is a degree of bubble size corresponding to putrefaction risk period, care period or safe period.

Also, the invention proposes an indicator for evaluating a quality of foods and drinks in which a microorganism having a nature of producing a gas, i.e. an aerogen is filled and sealed in a closed transparent vessel or transparent soft film bag of a synthetic resin together with a substrate consisting of a culture fluid and/or a culture medium, for example, a fermentation substrate.

In the indicator according to the invention, the aerogen is any one of yeast, mold and bacterium, and produces CO₂ and H₂ accompanied with the formation of an acid mainly from a carbohydrate above a temperature starting the gas generation. Also, it is desirable that the microorganism derived from the foods and drinks is sealed together with components such as the foods and drinks to be judged. Further, it is preferable that a standard bubble size indicating a degree of quality is previously printed on a surface of the vessel or soft film bag of the synthetic resin and that the standard bubble size printed is a degree of bubble size corresponding to putrefaction risk period, care period or safe period.

### BRIEF DESCRIPTION OF THE DRAWINGS

FIG. 1 is a graph showing a relation between bubble size and time in Experiment 1-2.
FIG. 2 is a photograph showing a change of bubble size with the lapse of time (environment temperature: 5°C) in Experiment 1-2.
FIG. 3 is a photograph showing a change of bubble size with the lapse of time (environment temperature: 23°C) in Experiment 1-2.
FIG. 4 is a photograph showing an amount of gas generated with the lapse of time (environment temperature: 23°C) in Experiment 2-1.

### BEST MODE FOR CARRYING OUT THE INVENTION

Microorganisms playing an important role in the invention, particularly a microorganism having a gas producing function in the foods and drinks will be described. In general, the microorganism takes fermentation and putrefaction functions in the foods and drinks. The fermentation is a phenomenon that an organic substance is metabolized by useful microorganisms and enzymes produced therefrom to produce an useful substance, and is widely used in fields of brewing or fermentation foods (soy source, soybean paste and the like). On the contrary, the putrefaction means a phenomenon that harmful microorganisms (putrefactive microorganism) excrete off enzymes decomposing carbohydrate, protein, fats and the like in the foods outside fungal form and breed while using the decomposed product as a nutrition source. Moreover, the decomposed product (organic acid, aldehyde, ammonia and the like) causes putrefied odor, change of taste, discoloration and the like.

Now, the gas generating function of the microorganism means a function that the microorganism generates gases such as CO₂, H₂ and the like together with the decomposed product when the organic substance in the foods is decomposed by metabolism or the enzyme produced. The microorganism having such a function is called as an aerogen. The decomposition of the organic substance by the aerogen starts about a temperature exceeding the gas-generating start temperature, and a small amount of the gas is gradually generated from the increased aerogens, respectively. Moreover, the gas-generating start temperature differs in accordance with the kind of the aerogen or the stored state, but is within a range of 1-10°C in almost of aerogens.

Moreover, the microorganism is a general term of small living things capable of being first observed by a microscope and includes yeasts, molds, bacteria and so on. In Table 1 are shown kind of main organic fermentation, microorganisms conducting fermentation and main microorganisms accompanied with fermentation.

**[Table 1]**

| | Microorganisms | Decomposed product |
|---|---|---|
| Fermentation through alcohol | · yeast (*Torulopsis,* Candida, etc.) | ethanol, CO₂ |
| | · molds | |
| | · a part of bacteria (*Zymomonas*, etc.) | |
| Fermentation through hetero type lactic acid | · a part of *Leuconostoc* and *Lactobacillus* (*Leu*.*meseateroides*, etc.) | lactic acid, acetic acid, formic acid, ethanol, CO₂ |
| Fermentation through mixed acids | · *E*.*coli*, Salmonella, *Erwinia*, etc. | lactic acid, ethanol, acetic acid, H₂, CO₂ or formic acid |
| Fermentation through butylene glycol | · *Elebsiella*, *Bacillus polymyxa*, etc. | 2,3-butylene glycol, lactic acid, ethanol, acetic acid, H₂, CO₂ or formic acid |
| Fermentation through butyric acid and butanol-acetone | · a part of *Clostridium* | butyric acid, acetic acid, H₂, CO₂, butanol, ethanol, acetone, isopropanol |
| | · *Bacillus macerans* | |
| Fermentation through propionic acid | · *Propionibacterium* and its closely related anaerobe | propionic acid, acetic acid, CO₂ |

The invention proposes a method of evaluating a quality (freshness) of the foods and drinks by utilizing such a microorganism, particularly a gas-generating function of an aerogen as well as an indicator used in this method. As mentioned above, the decomposition reaction of the organic substance by the aerogen, i.e. the gas-generating reaction gradually proceeds in accordance with the environmental temperature storing the foods and drinks and the time of passage (keeping). Also, it is influenced by the presence or absence of water, oxygen and nutrient, pH value and the like, so that an indicator is prepared by adjusting a fermentation substrate containing the aerogen (sample liquid) to the same condition as the foods and drinks and filling and sealing it into a vessel made of a synthetic resin or a small bag formed by heat sealing a soft film. Further, the indicator is placed and stored under the same environment as the foods and drinks to measure a degree of a bubble size through a gas generated in the indicator, whereby the quality of the foods and drinks can be judged. In this case, it is desirable that the aerogen resulted from the foods and drinks to be judged is sealed into the small bag, but it is not necessarily limited to such an aerogen. It is more desirable that the component of the foods and drinks is sealed as a substrate. However, there is an affinity between the substrate and the aerogen, so that it is preferable to select such a combination in accordance with the objective to be judged.

The evaluating method according to the invention lies in a point that the same reaction as the decomposition reaction through the microorganism resulted in the factor of the putrefaction of the foods and drinks can be recreated in the small bag, which is superior to the conventional technique. For example, when the indicator according to the invention is kept under the same environment together with the foods and drinks in the distribution course, the degree of the putrefaction reaction through the microorganism in the foods and drinks can be directly reproduced in the indicator and can objectively caught by visual perception, and hence the quality of the foods and drinks (freshness, degree of putrefaction or the like) can be indicated more precisely.

As the aerogen used in the evaluating method and the indicator according to the invention, it is preferable to use one or more of microorganisms shown in Table 2.

**[Table 2]**

| | |
|---|---|
| Yeast | *Saccharomyces* genus |
| | *Zygosaccharomyces* genus |
| | *Torulaspora* genus |
| | *Candida* genus etc |
| Mold | *Aspergillus* genus |
| | *Penicillium* genus |
| | *Mucor* genus etc |
| Bacteria | *Leuconostoc* genus: |
| | *Leu.mesenteroides* |
| | *Leu*.*paramesenteroides* |
| | *Leu.lactis* |
| | *Leu.oenos* etc |
| | *Lactobacillus* genus: |
| | *L*.*pentosus* |
| | *L.suebicus* |
| | *L*.*collinodes* |
| | *L*.*buchneri* |
| | *L.brevis* |
| | *L*.*fermentum* |
| | *L*.*kefir* |
| | *L*.*kandleri* |
| | *L*.*confuses* |
| | *L*.*sanfrancisco* |
| | *L.minor* |
| | *L*.*halotolerans* |
| | *L.viridescens* |
| | *L*.*fructivorans* |
| | *L*.*reuteri* |
| | *L*.*oris* |
| | *L*.*Ailgardi* |
| | *L*.*vaccinestercus* etc |
| | *Bifidobacterium* genus |
| | *B*.*pseudocatenulatum* |
| | *B*.*dentium* |
| | *B*.*adolescentis* |
| | *B*.*animalis* |
| | *B*.*gallinarum* |
| | *B*.*pscudolongum* |
| | *B*.*longum* |
| | *B*.*pullorum* |
| | *B*.*catenulateum* |
| | *B*.*angulatum* |
| | *B*.*globosum* |
| | *B*.*magnum* |
| | *B*.*coryneforme* |
| | *B*.*asteroids* |
| | *B*.*suis* |
| | *B*.*cuniculi* |
| | *B*.*infantis* |
| | *B.breve* |
| | *B*.*indicum* |
| | *B.subtile* |
| | *B*.*thermophilum* |
| | *B*.*boum* |
| | *B.minimum* |
| | *B*.*bifidum* |
| | *B*.*cboerinum* etc |

Among the aerogens shown in Table 2, *Aspergillus* genus mold and *Lactobacillus* genus bacteria and the like are adaptable in the evaluating method of the invention, and it is particularly preferable to use *Saccharomyces* genus yeast. Moreover, *Saccharomyces* genus yeast is a microorganism usually used in the production of breads and beer, and *Aspergillus* genus mold is a microorganism used in the production of refined sakes, and *Lactobacillus* genus bacteria are microorganisms used in the production of yoghurts and cheese, so that these microorganisms can be used safely.

As the *Saccharomyces* genus yeast, there are particularly a bread yeast, brewage yeast, feed yeast, yeast of raw material for nucleic acid and the like, which are also existent in substrates having a high molasses density such as fruit juice, fruit skin, sap or the like in addition to brewed products. Moreover, as the bread yeast is typically mentioned Saccharomyces *cerevisiae*, which includes freeze-resistant yeast and refrigeration resistant yeast not losing the activity even in freezing treatment-refrigeration storage of bread dough. The general bread yeast may ferment at 4°C, while the refrigeration-resistant yeast is characterized by fermenting at 10°C and also the freeze-resistant yeast is characterized by hardly bringing about the lowering of fermentation performance through the freezing.

Moreover, the yeasts conducts fermentation through alcohol under anaerobic conditions, which decomposes a nutrient such as sugar (glucose) or the like to produce carbon dioxide and ethanol. On the other hand, they conducts respiration under aerobic conditions and proliferate at a proper temperature around 30°C and pH: 5-6 in a liquid containing a nutritious source as shown in Table 3.

**[Table 3]**

| Nutritious source | |
|---|---|
| Carbon source | organic carbon source: glucose, fructose, saccharose, maltose, etc. |
| Nitrogen source | inorganic nitrogen: ammonium salt |
| | organic nitrogen: urea, amino acid, peptone, yeast extract, etc. |
| Inorganic salt | a small amount of inorganic salt is required. In this case, P, K, Mg, S are more required as compared with the other elements. |
| Growth factor | vitamin B1, biotin, inositol, nicotinic amide, pantothenic acid, pyridoxin and the like may be required |

The preparation method of the indicator according to the invention will be described below.

At first, at least one of the aforementioned microorganisms is mixed with a culture liquid as a fermentation substrate and/or a culture medium for fermentation to prepare a sample liquid. Also, the microorganism and the fermentation substrate consisting of the culture liquid and/or culture medium are stored separately and mixed just before the filling into the small bag formed by heat-sealing length and breadth of the vessel of synthetic resin or the soft film.

Moreover, as an example of the culture liquid are preferably used one or more of milk, meat extract, fruit juice, vegetable juice, fermentation seasoning and the like. As an example of the culture medium are preferably used sugar content of a fruit such as grape or the like, sugar content of vegetable and the like, because there is a phenomenon that the sugar content is metabolized to alcohol and carbon dioxide gas, and further apple, potato and the like having a large sugar content are preferably used.

The thus prepared sample liquid (fermentation substrate) provided with the aerogen is filled and sealed in the transparent vessel of the synthetic resin or the small bag formed by heat-sealing the transparent soft film so as to see the content thereof to prepare an indicator. The filling and sealing method is carried out by using a liquid filling apparatus proposed in Japanese Patent No. 2930515, JP-A-2001-335005, JP-A-2002-2601 and the like, and in this case it is important to take a care so that air is not incorporated into the indicator. Since the method according to the invention is a technique of evaluating the degree of putrefaction by an amount of the gas produced in the indicator, when air is first incorporated into the indicator, the amount of the gas produced cannot be confirmed accurately.

In this meaning, it is a condition that the microorganism used in the invention is a slight aerobic bacteria, facultative anaerobic bacteria or anaerobic bacteria capable of growing even at a low oxygen concentration among the aerogens.

The indicator formed by filling and sealing the sample liquid containing the aerogen is refrigeration-stored below a gas-producing start temperature of the aerogen, i.e. at a temperature of not higher than 0°C prior to the use.

As the soft film bag of the synthetic resin adaptable for the invention are used plastic films of polyester, nylon, polypropylene and the like. Also, in order to ensure the safety, a material having a high strength such as biaxially oriented nylon of 25 µm or the like can be used as a base film for the film bag.

Also, it is preferable that a standard bubble size indicating the quality in accordance with the kind of the foods and drinks to be objected and the kind of the aerogen is previously printed on the surface of the indicator. The standard bubble size indicates magnifications of the bubble size corresponding to a risky stage, care stage and safe stage of the foods and drinks, respectively. When such a standard bubble size is previously printed, it is a merit that the food keeper can easily and visually confirm the quality (proceeding degree of putrefaction) without requiring the measurement of the bubble size every time.

Moreover, according to the inventors' studies, it has been found that all kinds of the bacteria to be sealed in the small bag or the like changes the gas generating time through the change of the bacteria temperature and also the final gas generating amount changes through the change of the substrate, e.g. sugar concentration. By utilizing these natures are optionally controlled the gas generating time and amount in the small bag, whereby it is possible to adjust the time in accordance with the use application and produce a product not breaking after the gas generation.

### EXAMPLES

### <Example 1>

The presence or absence of gas-generating reaction at keeping time and temperature and the bubble size are confirmed by using various foods and drinks containing microorganisms to select a food most suitable as a sample liquid for an indicator.

### (Sample to be tested)

Substrates (sample liquids) used in this experiment are shown in Table 4.

**[Table 4]**

| No. | Kind of product | Culture liquid, culture medium |
|---|---|---|
| 1 | milk | protein in milk (stock solution) |
| 2 | fermented milk 1 | protein in milk (stock solution) |
| 3 | fermented milk 2 | protein in milk (stock solution) |
| 4 | lact ice | protein in milk (stock solution) |
| 5 | dairy product: lactobacillary beverage | protein in milk (stock solution) |
| 6 | silk soybean curd | soy protein (soup · soybean curd) |
| 7 | rice bean paste | soy protein (stock solution · 10 times dilution) |
| 8 | salted food 1 | sugar content in vegetable (pickled liquid) |
| 9 | salted food 2 | sugar content in vegetable (pickled liquid) |
| 10 | salted food 3 | sugar content in vegetable (pickled liquid) |
| 11 | soy sauced food 1 | sugar content in vegetable (pickled liquid) |
| 12 | soy sauced food 2 | sugar content in vegetable (pickled liquid) |
| 13 | yellow pickled radish | sugar content of radish and rice bran (pickled liquid) |
| 14 | food pickled in vinegar | sugar content in vegetable (pickled liquid) |

### (Preparation of indicator for experiment)

An indicator for experiment is prepared by filling 2 ml of each product shown in Table 4 as a sample liquid into the small bag prepared by using a laminate film of NY¹⁵/XA-S⁵⁰ and then subjecting to heat sealing so as not to incorporate air therein.

### (Experiment 1-1)

The indicator filled with the each sample liquid (small bag) is left to stand in a constant temperature bath set to 25°C for 24 hours to determine the presence or absence of gas generated in the bag. The results are shown in Table 5. As seen from the results, the generation of gas is observed in the indicators of Nos. 7-10. Particularly, the generation of a great amount of gas is observed in the indicator of No. 9.

**[Table 5]**

| No. | Kind of product | Gas generation |
|---|---|---|
| 1 | milk | × |
| 2 | fermented milk 1 | × |
| 3 | fermented milk 2 | × |
| 4 | lact ice | × |
| 5 | dairy product : lactobacillary beverage | Δ |
| 6 | silk soybean curd soup soybean curd | × × |
| 7 | rice bean paste stock solution 10 times dilution | Δ ○ |
| 8 | salted food 1 | ○ |
| 9 | salted food 2 | ⓞ |
| 10 | salted food 3 | ○ |
| 11 | soy sauced food 1 | Δ |
| 12 | soy sauced food 2 | × |
| 13 | yellow pickled radish | × |
| 14 | food pickled in vinegar | × |
| ⓞ : presence of gas generation (large) | | |
| ○ : presence of gas generation | | |
| Δ : presence of gas generation (slight) | | |
| × : absence of gas generation | | |

Now, the gas generation reaction of microorganism is confirmed by using the indicator of No. 9 observing the most remarkable gas generation and changing the temperature.

### (Experiment 1-2)

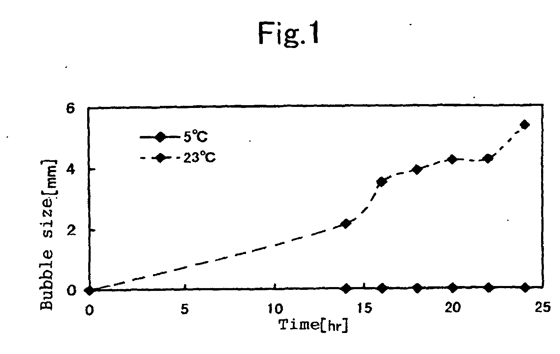

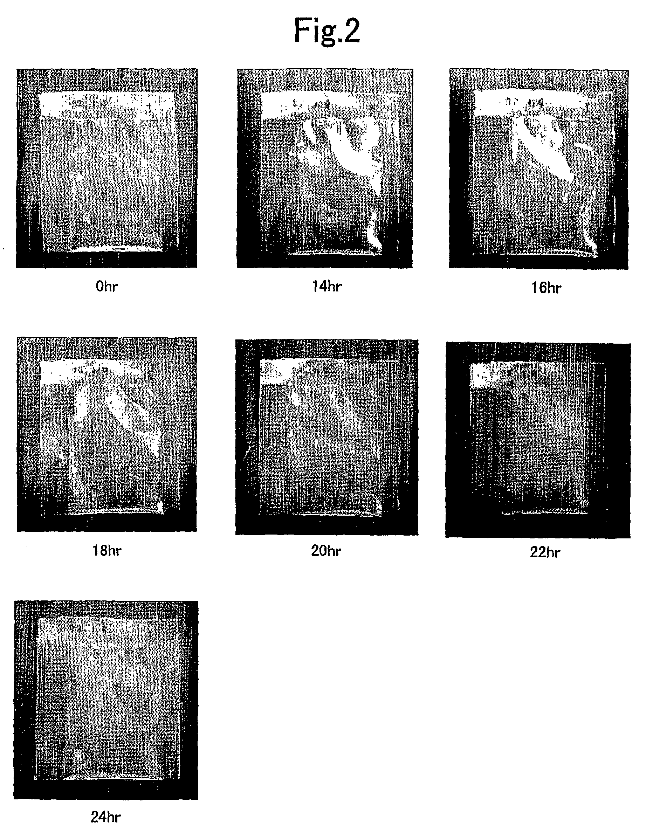

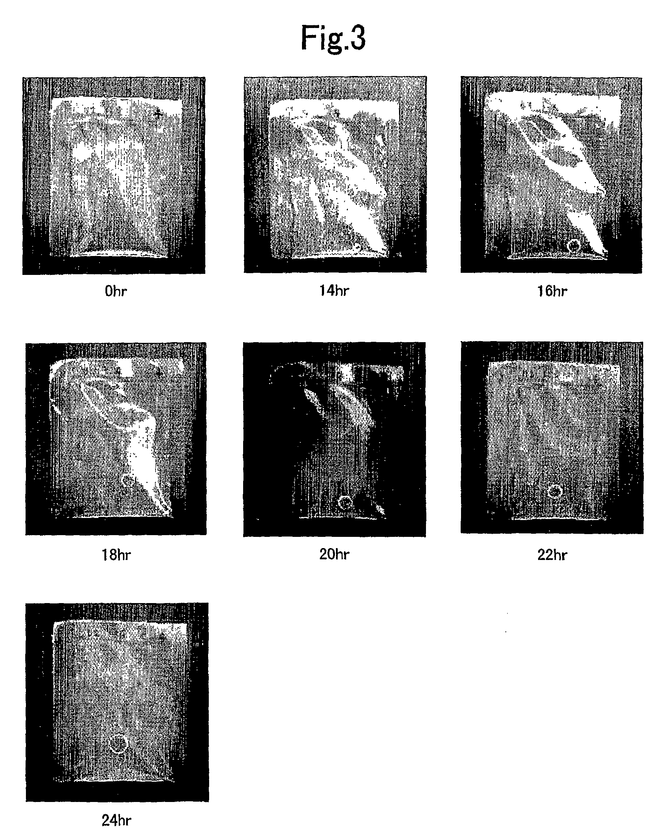

The indicator for experiment is left to stand in a room kept at 23°C or in a refrigerator of 5°C for 24 hours, and the bubble size generated in the indicator is measured three times after 14 hours of the standing and every 2 hours thereafter. The results are shown in Table 6 and FIG. 1. Moreover, FIG. 1 shows an average value of three measured results. Also, photographs taken every the measuring time are shown in FIGS. 2 and 3.

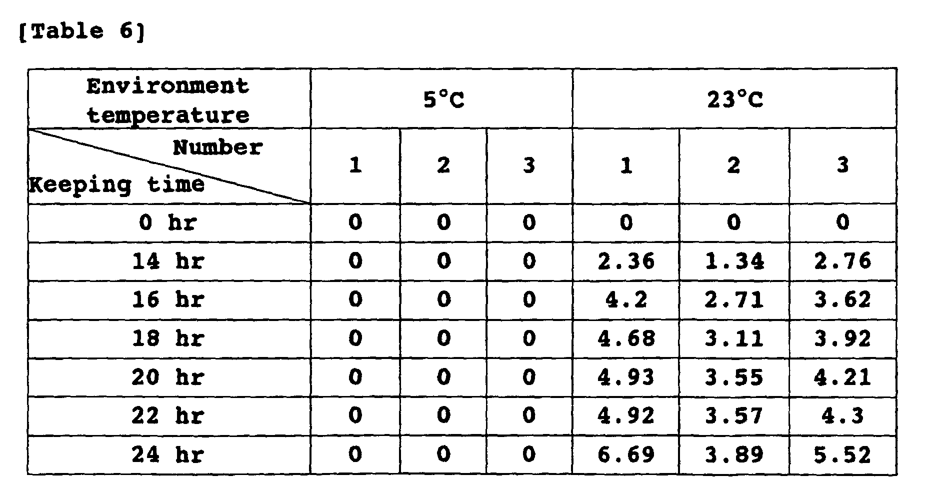

As seen from the results of Experiment 1-2, the generation of the bubbles is not observed under an environment of 5°C even after the standing for 24 hours, and hence the reaction through the aerogen does not proceed at a low temperature. However, the generation of the gas is observed under an environment of 23°C after 14-16 hours, and the bubble size becomes larger with the lapse of time.

### <Example 2>

The following experiment is carried out in order to confirm the effectiveness of a yeast as an aerogen used in the indicator according to the invention.

### (Preparation of indicator for experiment)

At first, a yeast solution is prepared by suspending 1 g of a yeast into 1 ml of a distilled water. A sample liquid is obtained by mixing 0.2 ml of the yeast solution with 10 ml of 5% glucose solution (culture liquid) and 1 ml of the sample liquid is filled in a small bag made of a laminate film of Ny/VM-PET/XA-S and heat-sealed so as not to incorporate air to prepare an indicator for experiment.

### (Experiment 2-1)

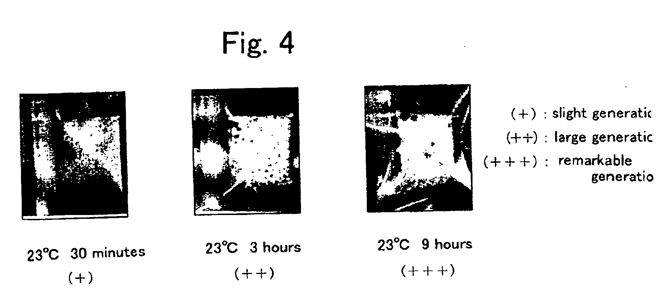

The thus prepared indicator (small bag) is left to stand in a constant temperature bath set to 23°C, 10°C, 4°C, 2°C and 0.5°C, respectively, to measure a time until glucose in the sample liquid is decomposed accompanied with alcohol fermentation through the yeast to produce CO₂. The results are shown in Table 7. In FIG. 4 is shown a photograph when the indicator is left to stand in a constant temperature bath of 23°C for 30 minutes, 3 hours or 9 hours. From the results of Table 7 and FIG. 4, it is confirmed that as the temperature becomes higher, the time required for alcohol fermentation becomes shorter, and CO₂ starts to produce at 23°C after 30 minutes and a greater amount of gas is produced so as to break the bag after 9 hours.

**[Table 7]**

| Temperature of constant temperature bath | Time required for producing gas |
|---|---|
| 23° | 30-60 minutes |
| 10°C | 4-5 hours |
| 4°C | 9-13 hours |
| 2°C | 64 hours |
| 0.5°C | 78-80 hours |

### (Experiment 2-2)

Then, there are examined changes of sugar concentration and living bacteria number in the sample liquid accompanied with the gas producing reaction. With respect to the above three indicators, the pH value, sugar concentration and living bacteria number are measured just after the preparation of the indicator and after being left to stand in a constant temperature bath of 23°C for 24 hours to obtain results shown in Table 8.

**[Table 8]**

| | | pH | sugar concentration(%) | Living bacteria number (CPU/ml) |
|---|---|---|---|---|
| Indicator 1 | just after the preparation of indicator | 3.94 | 5 | 1.4×10⁸ |
| | after the standing at 23°C for 24 hours | 3.38 | 2.5 | - |
| Indicator 2 | just after the preparation of indicator | 4.06 | 5 | 1.2×10⁸ |
| | after the standing at 23°C for 24 hours | 3.37 | 2.3 | 3.0×10⁷ |
| Indicator 3 | just after the preparation of indicator | 3.78 | 5 | 1.2×10⁸ |
| | after the standing at 23°C for 24 hours | 3.20 | 1.2 | 5.8×10⁷ |

As seen from the results of Table 8, in all of the indicators, the sugar concentration and pH value after the sample liquid is left to stand for 24 hours are lowered as compared with those just after the preparation of the indicator. This is due to the fact that glucose (sugar content) in the sample liquid is decomposed by alcohol fermentation through the yeast to produce CO₂ (acidity) as a decomposition product. Also, the living bacteria number decreases after being left to stand for 24 hours, which is considered due to the fact that glucose (sugar content) as a nutrient for the bacteria is consumed to cause abnormal metabolization of the bacteria (denutrition).

### (Experiment 2-3)

The aforementioned indicator for experiment (small bag) is frozen in a refrigerator of -22°C for 24 hours and defrost and left to stand in a constant temperature bath set to 23°C, 10°C and 4°C, respectively, to measure an amount of gas generated and a time required for alcohol fermentation. The results are shown in Table 9.

**[Table 9]**

| unit: hours | | | | |
|---|---|---|---|---|
| Temperature of constant temperature bath | | Amount generated (+)* | Amount generated (++)* | Amount generated (+++)* |
| 23°C | yeast 1 | 0.3 | 1.25 | 3 |
| | yeast 2 | 0.5 | 1.75 | 3 |
| 10°C | yeast 1 | 4 | 6 | - |
| | yeast 2 | 25 | - | - |
| 4°C | yeast 1 | - | < 15 | ≤ 87 |
| | yeast 2 | > 135 | ≤ 255 | - |

| | | | | |
|---|---|---|---|---|
| * (+) :slight generation, (++) : large generation, (+++) : remarkable generation | | | | |

From the results of Table 9, it is confirmed that even in all of the indicators, some times is required up to the gas generation as compared with the case of conducting no freezing-defreezinge treatment(Experiment 2-1, Table 7), but a large change in the gas generation is not observed and hence the yeast used in this experiment is hardly subjected to an obstruction through freezing (freeze-resistant yeast). Such a yeast can be expected to use, for example, as an indicator for frozen foods.

As seen from the results of Example 1, the indicator according to the invention is very useful when the quality of the food (proceeding degree of putrefaction) is judged in the indicator using at least the exemplified pickled liquid (culture liquid: sugar content of vegetable) though the long time is taken until the generation of the gas. Also, it is considered that the other non-sterilized foods (including vegetables and fruits) not used in this experiment can be utilized as the indicator of the invention. Furthermore, commercial products may be used as a sample for the indicator as they are, or various indicators can be provided in accordance with the temperature and the kind of the food by examining the kind of aerogen and the culture liquid (medium).

From the results of Example 2, it is confirmed that the yeast can be preferably utilized as an aerogen suitable for the indicator according to the invention. Also, since the fermentation temperature differs in accordance with the kind of the yeast, it is confirmed that when the yeast is properly selected in accordance with the food to be objected, it may be an excellent indicator.

### INDUSTRIAL APPLICABILITY

As mentioned above, according to the method of evaluating foods and drinks according to the invention and the indicator used for carrying out this method, the quality of the foods and drinks, particularly the degree of quality change dependent upon both the temperature history and the passage time can be judged accurately and simply in the form of recreating the quality change of the foods and drinks in the small bag. In the invention, the foods and drinks mean fresh foods such as farm products, livestock products, fish and shellfish and the like; processed foods such as packed lunch, dishes and the like; drinks such as vegetable juice, fruit juice, alcohol and the like; seasonings such as source, fermented soybean paste and the like; food oil, sample for biochemistry, cosmetics, industrial chemicals, medicines such as vaccine and the like; articles mainly causing quality change or putrefaction under an influence of temperature of keeping environment and keeping time such as blood, internal organs, culture medium for growth, seeds and the like. The invention is effective for evaluating the quality of such foods and drinks.

## Claims

1. A method of evaluating a quality of foods and drinks, **characterized in that** a microorganism of a nature producing a gas is sealed in a closed transparent vessel or transparent soft film bag of a synthetic resin and a quality of foods and drinks to be judged is judged by an amount of gas produced in the vessel or bag.

2. A method of evaluating a quality of foods and drinks according to claim 1, wherein the microorganism of a nature producing a gas is an aerogen.

3. A method of evaluating a quality of foods and drinks according to claim 2, wherein the aerogen is any one of yeast, mold and bacterium.

4. A method of evaluating a quality of foods and drinks according to claim 2 or 3, wherein the aerogen produces CO₂ and H₂ accompanied with the formation of an acid mainly from a carbohydrate above a temperature starting the proliferation.

5. A method of evaluating a quality of foods and drinks according to claim 1, wherein a standard bubble size indicating a degree of quality is previously printed on a surface of the vessel or soft film bag of the synthetic resin.

6. A method of evaluating a quality of foods and drinks according to claim 5, wherein the standard bubble size printed is a degree of bubble size corresponding to putrefaction risk period, care period or safe period.

7. A method of evaluating a quality of foods and drinks according to claim 1, wherein the microorganism is a microorganism derived from the foods and drinks to be judged and is sealed in the vessel or the bag.

8. An indicator for evaluating a quality of foods and drinks in which a microorganism having a nature of producing a gas is filled and sealed in a closed transparent vessel or transparent soft film bag of a synthetic resin together with a substrate.

9. An indicator for evaluating a quality of foods and drinks according to claim 8, wherein the microorganism of a nature producing a gas is an aerogen and the substrate is a culture fluid and/or a culture medium of the aerogen.

10. An indicator for evaluating a quality of foods and drinks according to claim 9, wherein the aerogen is any one of yeast, mold and bacterium.

11. An indicator for evaluating a quality of foods and drinks according to claim 9 or 10, wherein the aerogen produces CO₂ and H₂ accompanied with the formation of an acid mainly from a carbohydrate above a temperature starting the proliferation.

12. An indicator for evaluating a quality of foods and drinks according to claim 8, wherein a standard bubble size indicating a degree of quality is previously printed on a surface of the vessel or soft film bag of the synthetic resin.

13. An indicator for evaluating a quality of foods and drinks according to claim 12, wherein the standard bubble size printed is a degree of bubble size corresponding to putrefaction risk period, care period or safe period.

14. An indicator for evaluating a quality of foods and drinks according to claim 8, wherein the microorganism is a microorganism derived from the foods and drinks to be judged and is sealed in the vessel or the bag.
